Europäisches Patentamt

European Patent Office

Office européen des brevets

(11) Veröffentlichungsnummer: **0 121 729**
**B1**

(12) **EUROPÄISCHE PATENTSCHRIFT**

(45) Veröffentlichungstag der Patentschrift :
09.08.89

(51) Int. Cl.⁴: **C 12 M   1/00**

(21) Anmeldenummer : 84102144.7

(22) Anmeldetag : 29.02.84

(54) Biohochleistungsdurchlaufreaktor.

(30) Priorität : 08.03.83 DE 3308173

(43) Veröffentlichungstag der Anmeldung :
17.10.84 Patentblatt 84/42

(45) Bekanntmachung des Hinweises auf die Patenterteilung : 09.08.89 Patentblatt 89/32

(84) Benannte Vertragsstaaten :
AT BE CH DE FR GB IT LI LU NL SE

(56) Entgegenhaltungen :
AT–B–   369 782
DE–A– 3 102 739
DE–A– 3 214 798
DE–A– 3 232 530
US–A– 4 336 135

(73) Patentinhaber : Schraufstetter, Wilfried
Schlossstrasse 5
D-8045 Ismaning (DE)

(72) Erfinder : Schraufstetter, Wilfried
Schlossstrasse 5
D-8045 Ismaning (DE)

(74) Vertreter : Lesser, Karl-Bolko, Dipl.-Ing.
European Patent Attorney Johanneskirchnerstrasse
149a
D-8000 München 81 (DE)

## Beschreibung

Die Erfindung betrifft einen Biohochleistungsdurchlaufreaktor gemäß Oberbegriff des Anspruches 1.

Stand der Technik

Biohochleistungsdurchlaufreaktoren dienen zur anaeroben Aufbereitung pflanzlicher und tierischer Biomasse, insbesondere Abfallmasse, aus organischen Materialien mit einem relativ hohem Molekulargewicht. In dem Reaktorraum des Reaktors werden die hochmolekulären organischen Materialien durch Bakterienstämme in organische Materialien mit niedrigem Molekulargewicht und diese in niedrige organische Säuren unter Bildung von Wasserstoff, Kohlendioxid und Schwefelwasserstoff in Essigsäure übergeführt. Die entstehenden Substanzen werden dann in Methan und Kohlendioxid umgewandelt, wobei in dem Reaktor eine Aufteilung von Säurephase und Methanphase, vorzugsweise im umgekehrten Verhältnis zu den Abbauraten der Prozeßstufen Säurebildung und Methanbildung, erfolgt. Insbesondere die in der Säurephase abgebauten Produkte (im wesentlichen Essigsäure und Wasserstoff gelangen dann in einen Fermentationsraum für die Methanbildung.

Ein solcher Reaktor ist aus der deutschen Offenlegungsschrift DE-A-3.102.739 bekannt. Diese bekannten, aus Stahl hergestellten Reaktoren weisen in ihrem Deckel über die Fläche desselben verteilt mehrere Öffnungen auf, um die Biomasse zu- und abzuführen und um die entstandenen Gase abzuführen. Jeder dieser Öffnungen ist ein Rohrstutzen zugeordnet, an denen Zu- und Abführleitungen festgelegt sind.

Gemäß einem vorbekannten Verfahren wird die Biomasse oben durch ein Einfüllrohr in einen Reaktor gegeben, wobei in diesem Einfüllrohr, welches bis fast auf den Boden des Reaktors reicht, die Säurebildung stattfindet. Nach der Säurebildung, bei der ein Teil der gasförmigen Produkte nach oben aufströmt, wird die Masse in den Fermentationsraum weitergeleitet, in dem die Methanbildung stattfindet. Dieser Fermentationsraum, der durch die metallenen Reaktorwände und die Wandung des metallenen Einfüllrohres begrenzt ist und der über am unteren Ende des Einfüllrohres vorgesehene Öffnungen mit dem Inneren des Einfüllrohres verbunden ist, findet die Methanbildung statt, wobei sowohl das gebildete Methan, als auch die verbleibenden Substanzen oben abgeführt werden. Da die Bakterien in diesem bekannten Reaktor nicht immobilisiert vorliegen, kann in diesem Reaktor, um einen großen Bakterienverlust zu vermeiden, nur mit einem kleinen Durchsatz pro Zeiteinheit gefahren werden, dementsprechend liegt die Verweilzeit der Masse etwa bei fünf Tagen.

Aus dem US-Patent US-A-4.321.141 ist ein ähnliches Verfahren bekannt, wobei dort die Säurebildung und die Methanbildung in zwei miteinander verbundenen Reaktoren vorgenommen wird. Beide Reaktoren gemäß der US-A-4.321.141 sind mit einem offenporigem und porösem Material gefüllt, das eine spezifische Oberfläche von etwa 0,01 m²/g und einen Porendurchmesser von 0,8 µm bis 220 µm aufweist. Dieses bekannte Material kann aus silikatischen oder nichtsilikatischen Metalloxiden bestehen, die entweder amorph oder kristallin vorliegen. Mittels dieses bekannten porösen Materials lassen sich die Bakterienstämme immobilisieren, was zu einer kleineren Verweilzeit der abzubauenden Masse und damit zu einem größerem Durchsatz bezogen auf das Reaktorvolumen führt.

Das für die Reaktoren der US-A-4.321.141 benutzte Material liegt dort in Form von extrudiertem Granulat mit einem Durchmesser von ungefähr 2 mm und mit einer Länge von 2 bis 6 mm vor. Als Material wurde z. B. Cordierit mit einer durchschnittlichen Porengröße von 3 µm bei einem Porengrößenbereich von 2 µm bis 9 µm benutzt. Der dabei verwendete zweite Reaktor, in dem die anaerobische Reaktion stattfindet, besteht dort aus einem Glasrohr, dessen Längsachse mit der Horizontalen einen kleinen Winkel einschließt, also relativ flach liegt. Der dort verwendete Reaktor war 250 mm lang und hatte einen Durchmesser von 15 mm. Diese Reaktor ist mit dem offenporigem und porösem, anorganischem Material nur zum Teil gefüllt.

Bei der dort gezeigten und beschriebenen Vorrichtung handelt es sich um eine Versuchsanlage zur Ermittlung optimaler Ergebnisse. Dies läßt sich schon daraus entnehmen, daß der Flüssigkeitsdurchsatz zwischen etwa 4 und 50 ml/h, insbesondere 20 bis 40 ml/h, und das entstandene Gas zwischen 0 und 24 ml/h betrug. Als zugeführte Flüssigkeit wurde städtisches Abwasser benutzt, aus welchem zuvor Feststoffe mittels Geweben und Glaswolle entfernt wurden. Der Wasseranteil betrug mindestens 50 Gewichtsprozent und bevorzugt zwischen 80 und 98 Gewichtsprozent der zugeführten Flüssigkeit.

Um in einem Reaktor gemäß der US-A-4.321.141 Biogas herstellen zu können sind also besondere Vorbedingungen notwendig, insbesondere was die zugeführte Masse betrifft.

Feststoffe würden insbesondere zwischen den Granulaten des zweiten Reaktors und den Granulaten des ersten Reaktors, der die gleichen Granulate enthält, aber etwas anders und kleiner ausgebildet ist, hängenbleiben und den Durchfluß verhindern.

Solche mit offenporigem und porösem, granuliertem, anorganischem Material gefüllten Reaktoren sind somit nicht geeignet pflanzliche und tierische Biomasse, die auch aus festen und größeren Teilen besteht, wie z. B. Pflanzenstengel, Essensreste, Fleisch- und Knochenabfälle, sowie sonstige gegebenenfalls zerkleinerte Abfallmasse zu verarbeiten.

Die bei dem in der US-A-4.321.141 beschriebenen Verfahren benutzten Materialien sind auch sehr teuer, was sich bei der großtechnischen Nachbildung der Versuchsanlage als gewichtiger Kostenfaktor bemerkbar macht.

Ein Reaktor gemäß der US-A-4.321.141 hat andererseits den obenbeschriebenen großen Vorteil der kurzen Verweilzeit, die gegenüber dem anderen bekannten Reaktor je nach Biomasse zwischen zwei und fünf Stunden beträgt.

Große Reaktoren weisen inzwischen ein Füllvolumen von 1 000 m³ und mehr auf. Solche Großreaktoren werden dann aus statischen Gründen in der Regel aus Beton hergestellt. Zum Zuund Abführen der Biomasse haben solche bekannte Reaktoren mehrere im Deckel des Behälters angeordnete, voneinander beabstandete Öffnungen, durch die die Biomasse und die entstehenden Gase zu- und abgeführt werden. Die Biomasse bewegt sich dabei von einer Einlaßöffnung zu einer Auslaßöffnung. Dabei sind die Strömungsverhältnisse nicht ideal, d. h., ein Teil der Biomasse bewegt sich schneller von Öffnung zu Öffnung, wie ein anderer Teil der Biomasse, sodaß die Verweilzeit der Biomasse im Reaktor sehr unterschiedlich ist und eine optimale Aufarbeitung der Biomasse nicht ermöglicht wird. In bekannten Reaktoren befinden sich beim Betrieb im wesentlichen nur Biomasse und entstandene Gase. Setzt sich in einem solchen Reaktor ein Teil der Biomasse, die aus festen und flüssigen Bestandteilen besteht, fest, so ändern sich die Strömungsverhältnisse im Reaktor besonders stark, sodaß die Verweilzeit der Biomasse im Reaktor nicht kontrolliert werden kann. Insbesondere kommt es zur Bildung von kurzen Strömungskanälen, durch die die Biomasse auf dem kürzesten Weg den Reaktor wieder verläßt, ohne im wesentlichen abgebaut zu sein.

Ein weiterer Nachteil einer Vielzahl verschiedener Öffnungen mit zum Teil sehr großen Durchmessern im Deckel der bekannten Groß-Reaktoren besteht darin, daß dieser Deckel aus statischen Gründen wegen der Öffnungen eine sehr große Dicke aufweisen muß, die jetzt schon zwischen 0,4 und 0,45 m bei der Verwendung von Beton beträgt. Diese relativ große Wandstärke ist auch notwendig, da während der Durchführung des Verfahrens im Reaktor ein Überdruck herrscht.

Da bei den bekannten Reaktoren sämtliche Zuund Abführleitungen einzeln durch den Behälterdeckel oder auch zum Teil durch die Behälterwände hindurchgeführt sind, ist auch die Herstellung eines solchen Reaktors schwierig. Bei kleineren Reaktoren, insbesondere solchen aus Stahl, können zwar die einzelnen Rohrstutzen nach Herstellen jeweils einer Öffnung an dem Deckel oder der Wand angeschweißt werden ; bei größeren Reaktoren und insbesondere bei solchen aus Beton, können die Rohranschlüsse usw. erst am Einbauort mit dem Reaktorbehälter verbunden und gegenüber diesem abgedichtet werden.

Aufgabe der Erfindung

Der Erfindung liegt deshalb die Aufgabe zugrunde, einen gattungsgemäßen Biohochleistungsdurchlaufreaktor zu schaffen, in dem die Biomasse gleichmäßig geführt und abgebaut werden kann, um so eine möglichst kurze Verweilzeit der Biomasse in dem Reaktor und eine große Ausbeute an Biogas zu erhalten. Insbesondere aber soll der Reaktor einfach in Aufbau, Montage und Wartung sein.

Lösung der Aufgabe

Diese Aufgabe wird durch einen Reaktor gemäß Anspruch 1 gelöst. Bei dem erfindungsgemäßen Reaktor werden durch den neuen Einsatz nicht nur im wesentlichen sämtliche Zu- und Abführeinrichtungen für die Biomasse und die entstandenen Gase an einem vom Reaktorbehälter trennbaren Teil vereinigt, dieser Einsatz selbst weist darüberhinaus auch eine rohrförmige Einrichtung zum Aufteilen des Reaktorraumes in einen Säurebildungsraum und in einen Fermentationsraum auf.

Der Reaktorbehälter, bestehend aus einem Boden, Seitenwänden und einem Deckel, dessen Deckel, möglichst zentrisch, eine Öffnung besitzt, in die der Einsatz einhängbar ist, kann somit unabhängig von den anderen zum Betrieb notwendigen Bauteilen, je nach Größe z. B. aus Stahl oder Beton, hergestellt werden. Unabhängig davon kann der Einsatz gefertigt werden, der z. B. erst am Aufstellort des Reaktors in die Öffnung des Behälterdeckels eingesetzt wird. Ein z. B. zylinderförmig ausgebildeter Reaktor besitzt dann einen kreisförmigen Deckel, in dessen Zentrum die Öffnung angeordnet ist. Durch diese zentrische Anordnung der Öffnung kann der den Einsatz tragende Deckel des Reaktors dünner und damit auch leichter ausgebildet werden. Die zentrische Lage der Zu- und Abführeinrichtungen insbesondere für die Biomasse bringt nun den weiteren Vorteil, daß die Biomasse in dem Reaktor gleichmäßiger geführt werden kann.

Der erfindungsgemäße Einsatz besteht im wesentlichen aus mehreren zueinander konzentrisch angeordneten Rohren, deren gemeinsame Achse im eingebauten Zustand vertikal ausgerichtet ist und die Längsachse des Einsatzes bildet. Diese Rohre, die bevorzugt kreiszylinderförmig ausgebildet sind, sind über Flanschringe miteinander verbunden. Dadurch werden mehrere, voneinander abgetrennte Räume geschaffen. Der Säurebildungsraum, in den die frische Biomasse zuerst gelangt, wird dadurch gebildet, daß am oberen Ende eines ersten Rohres ein sich nach innen erstreckender erster Flanschring festgelegt, z. B. angeschweißt, ist, an dessem inneren Rand das untere Ende eines zweiten, kleineren Rohres angeordnet ist. Das zweite Rohr ist gegenüber diesem ersten Flanschring abgedichtet und kann gegenüber diesem Flanschring und damit gegenüber dem ersten Rohr axial verschoben werden. Durch diese Ausbildung kann nicht nur der obere Teil des Einsatzes, also der « Kopf » des Einsatzes, gegenüber dem unteren Teil des Einsatzes, der im wesentlichen aus dem ersten Rohr und dem im folgenden zu beschreibenden Mantel besteht, verschoben werden. Der Transport und der Ein- bzw. Ausbau des Einsatzes in den Reaktorbehälter wird dadurch erleichtert. Dieses zweite Rohr liegt mit dem größeren Teil seiner axialen Länge außerhalb des Reaktorbehälters. Während somit im Innern dieser beiden Rohre die Säurebildung stattfindet, findet außerhalb dieser beiden Rohre die Fermentation statt.

Das untere, erste und größere Rohr ist fast über seine ganze Länge von einem Mantel umgeben. Zwischen diesem Mantel und diesem Rohr wird ein hohlzylinderförmiger Raum gebildet, in dem eine Wärmeflüssigkeit fließt, die über zwei Leitungen zu- bzw. abgeführt wird. Diese Wärmeflüssigkeit dient dazu, die im Reaktor befindliche Biomasse auf der optimalen Verfahrenstemperatur von etwa 34 °C zu halten. Der Außendurchmesser des Mantels ist kleiner, als der Durchmesser der Öffnung des Reaktordeckels, sodaß der Einsatz von oben in die Öffnung z. B. mittels eines Kranes eingehängt werden kann.

Das zweite, kleinere Rohr ist von einem dritten Rohr umgeben und mit diesem über einen zweiten Flanschring verbunden, vorzugsweise verschweißt. In den Raum zwischen diesen beiden Rohren wird die abgebaute Biomasse geführt. In diesem Raum sammelt sich auch ein Teil des entstandenen Biogases, welches über einen an dem zweiten Flanschring angeordneten und in diesen Raum mündenden Rohrstutzen abgeführt werden kann. Der Außendurchmesser dieses dritten Rohres entspricht etwa dem Außendurchmesser des Mantels des ersten Rohres bzw. ist etwas größer als dieser und ist andererseits geringfügig kleiner, als der Durchmesser der Öffnung des Reaktordeckels. Das untere Ende des dritten Rohres ist von dem oberen Ende dieses ersten Mantels beabstandet, und zwar dadurch, daß der Durchmesser des dritten Rohres größer ist, als der Durchmesser des Mantels oder daß das dritte Rohr axial von dem Mantel des ersten Rohres beanstandet ist.

Das obere Ende des ersten, größeren Rohres ist dabei von der Unterkante des Reaktordeckels beabstandet, während das zweite, kleinere Rohr und das dritte Rohr sich durch die Öffnung des Reaktordeckels hindurcherstrecken und über diesen hinausstehen. Dadurch werden durch das Innere des zweiten Rohres und durch den Raum zwischen dem zweiten und dem dritten Rohr zwei Durchtrittsöffnungen durch den Reaktordeckel geschaffen, durch die zum einen frische Biomasse in den Reaktor eintreten und zum anderen ausgefaulte Biomasse aus dem Reaktor austreten kann.

Das dritte Rohr ist an seiner Außenseite gegenüber dem Deckel des Reaktors in der Deckelöffnung abgedichtet, sodaß weder Gas, noch Biomasse entweichen kann. Hierzu ist an der Außenseite des dritten Rohres ein Auflageflansch vorgesehen, vorzugsweise angeschweißt. Dieser Auflageflansch liegt auf einer Dichtung auf, die das dritte Rohr gegenüber der Öffnung des Deckels des Reaktorbehälters abdichtet.

Der untere Teil des Einsatzes steht mit dem unteren Ende des ersten, großen Rohres auf dem Boden des Reaktorbehälters auf, wo er gegen seitliches Verrutschen z. B. mittels eines am Boden des Behälters befestigten L-förmig profilierten Flanschringes oder mittels am Behälterboden befestigten Winkeln geführt sein kann.

Im mittleren Bereich des dritten Rohres und außerhalb des Reaktorbehälters befindet sich an dem dritten Rohr ein horizontal liegender dritter Rohrstutzen, über den die ausgefaulte Biomasse abgeführt werden kann.

Der Fermentationsraum kann in mehrere Kammern aufgeteilt sein, in die jeweils eine Leitung für die Zuführung der Biomasse mündet und die jeweils eine Einrichtung zum Abführen des Biogases und zum Abführen der ausgefaulten Biomasse aufweisen.

Der Einsatz kann aus Edelstahl, z. B. V2A-Stahl, oder auch aus Kunststoff bestehen. Die Blechstärke sollte dann zwischen 3 und 8 mm liegen. Der Reaktor arbeitet quasi-kontinuierlich, d. h. die mittlere Verweilzeit beträgt 24 Stunden, wobei z. B. jede Stunde frische Biomasse eingepumpt und ein Teil der Biomasse durch den Abführ-Rohrstutzen überläuft. Das Verfahren wird dabei vollautomatisch durchgeführt, wobei Schwimmerschalter im Einlauf- und Auslaufbereich dafür sorgen, daß im Notfall die Zufuhr frischer Biomasse gestoppt werden kann.

Kurzbeschreibung der Zeichnung

Weitere Einzelheiten und Vorteile der Erfindung, insbesondere die Aufteilung des Reaktors, die Führungen und das Füllgut betreffend, ergeben sich aus im folgenden anhand der Zeichnung beschriebenen Ausführungsbeispielen.

Es zeigt :

Figur 1 einen schematischen Schnitt durch einen Biohochleistungsdurchlaufreaktor mit einem Einsatz gemäß einer ersten Ausführungsform und einem Fermentationsraum in zwei verschiedenen Ausführungen ;

Figur 2 einen schematischen Schnitt durch den zentralen Teil eines Biohochleistungsdurchlaufreaktors und durch einen Einsatz in vergrößerter Darstellung gemäß einer zweiten Ausführungsform ;

Figur 3 einen schematischen Schnitt durch einen Biohochleistungsdurchlaufreaktor mit einem Einsatz gemäß einer dritten Ausführungsform ;

Figur 4 einen schematischen Schnitt durch den zentralen Teil des Biohochleistungsreaktors gemäß Figur 3 ;

Figur 5 einen Schnitt entlang der Linie V-V in Figur 3, und

Figur 6 eine Aufsicht auf den Reaktor gemäß Figur 3.

Beschreibung der bevorzugten Ausführungsbeispiele

Ein Biohochleistungsdurchlaufreaktor besteht im wesentlichen aus einem Reaktorbehälter 1, der einen flachen, horizontalen Boden 2, einen hier flachen und horizontalen Deckel 3 und eine zylinderförmige, senkrecht stehende Wandung 4 aufweist, und aus einem auf dem Boden 2 stehenden und durch eine Öffnung 5 des Deckels 3 dringenden Einsatz 6. Die vertikal stehende Achse 7 des Einsatzes 6 ist hier mit der vertikalen Achse des Reaktorbehälters 1 identisch.

Dieser Einsatz 6 besteht im wesentlichen aus mehreren konzentrisch zueinander angeordneten Rohren, deren Achsen in der Achse 7 des Einsatzes 6 liegen, wobei die Achse des Einsatzes 6 mit der Achse des Reaktorbehälters 1 zusammenfällt. Ein erstes, völlig im Innern des Reaktorbehälters 1 befindliches Rohr 8 trägt an seinem einen, oberen Ende einen sich nach innen erstreckenden Flanschring 9. Am Innenrand dieses Flanschringes 9 ist ein zweites Rohr 10 angeordnet, das einen entsprechend kleineren Durchmesser aufweist, als das zuvor beschriebene erste Rohr 8 und sich vom Flanschring 9 nach oben erstreckt. Während der Flanschring 9 am ersten Rohr 8 z. B. angeschweißt ist, kann das zweite Rohr 10 und damit der obere Teil des Einsatzes 6, also der « Kopf » des Einsatzes, gegenüber dem Flanschring 9 verschoben werden. Das durch den Flanschring 9 hindurchtretende zweite Rohr 10 ist gegenüber dem Flanschring 9 abgedichtet.

Die beiden Rohre 8 und 10, zwischen denen der Flanschring 9 angeordnet ist, umschließen einen Säurebildungsraum 12 des Reaktors und grenzen diesen gegenüber dem Fermentationsraum 13 des Reaktors ab.

Das erste, größere Rohr 8 ist von einem Mantel 14 umgeben. Zwischen diesem Mantel 14 und dem Rohr 8 wird ein Raum 15 gebildet, in dem eine Wärmeflüssigkeit strömt, die über Leitungen 16 und 17 zu- bzw. abgeführt wird. Diese Leitungen 16 und 17 verlaufen parallel zur Achse 7 des Einsatzes 6 und verlassen somit den Reaktor im Innern des Einsatzes 6. Die Wärmeflüssigkeit sorgt dafür, daß die in dem Reaktor befindliche Biomasse auf der optimalen Verfahrenstemperatur von etwa 34 °C gehalten wird. Der Außendurchmesser dieses Mantels 14 ist kleiner, als die Öffnung 5 des Reaktordeckels 3.

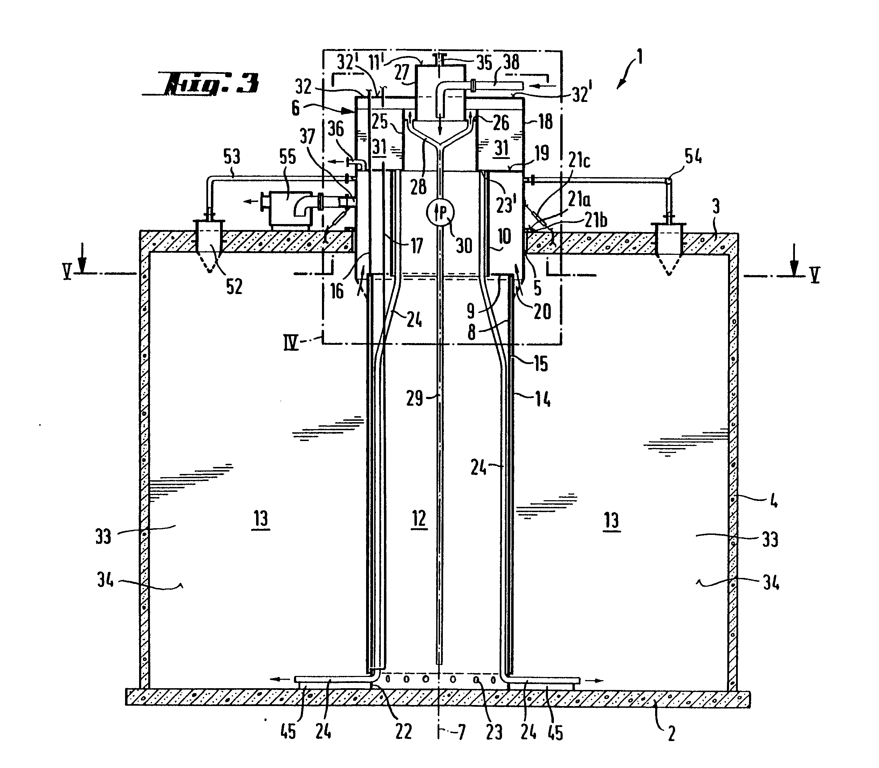

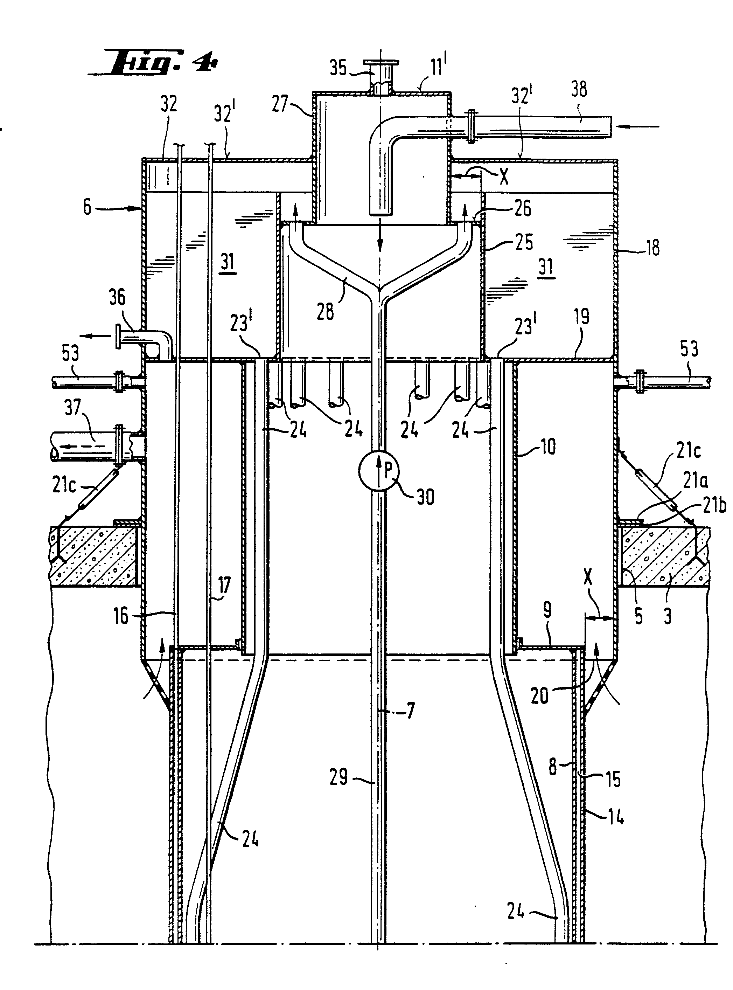

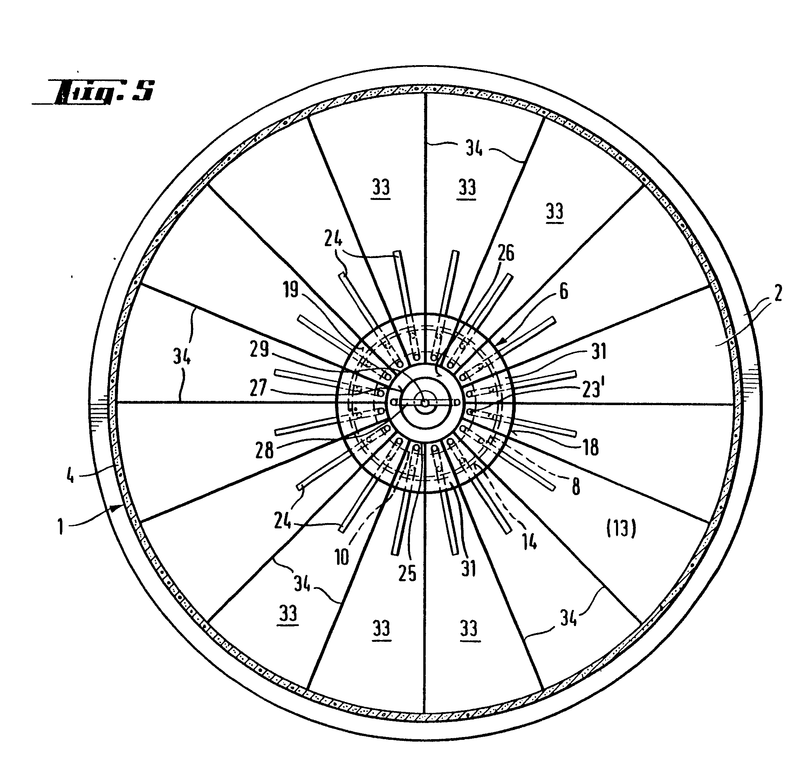

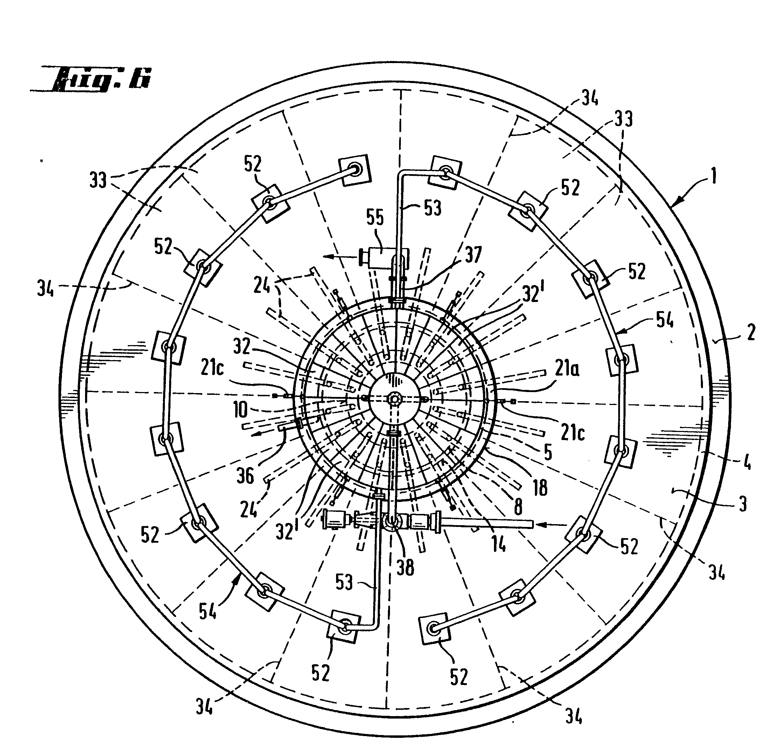

Der Mantel 14 reicht nun nicht bis an das untere Ende 22 des ersten Rohres 8, sondern endet etwas darüber. Dieses untere Ende 22 steht frei auf dem Boden 2 des Reaktorbehälters 1, es kann aber auch durch dort angeordnete, nicht dargestellte Winkel oder einen Flanschring, die am Boden 2 festgelegt sind, gegen seitliches Verschieben gehalten werden. Auch sollte mindestens bei der Ausführungsform nach Figuren 3 bis 6 der untere Rand des ersten Rohres 8 so auf dem Boden 2 des Reaktorbehälters 1 aufliegen, daß der Säurebildungsraum 12 gegenüber dem Fermentationsraum 13 abgedichtet ist.

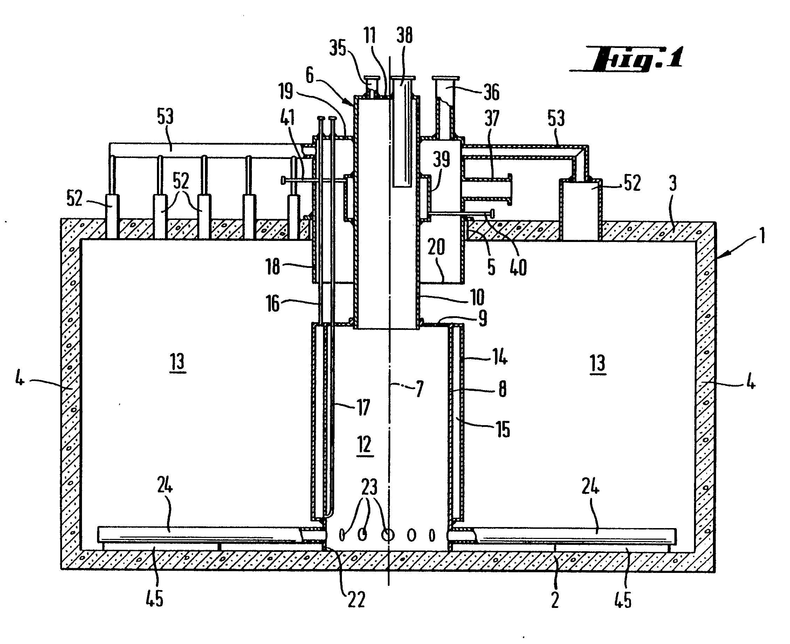

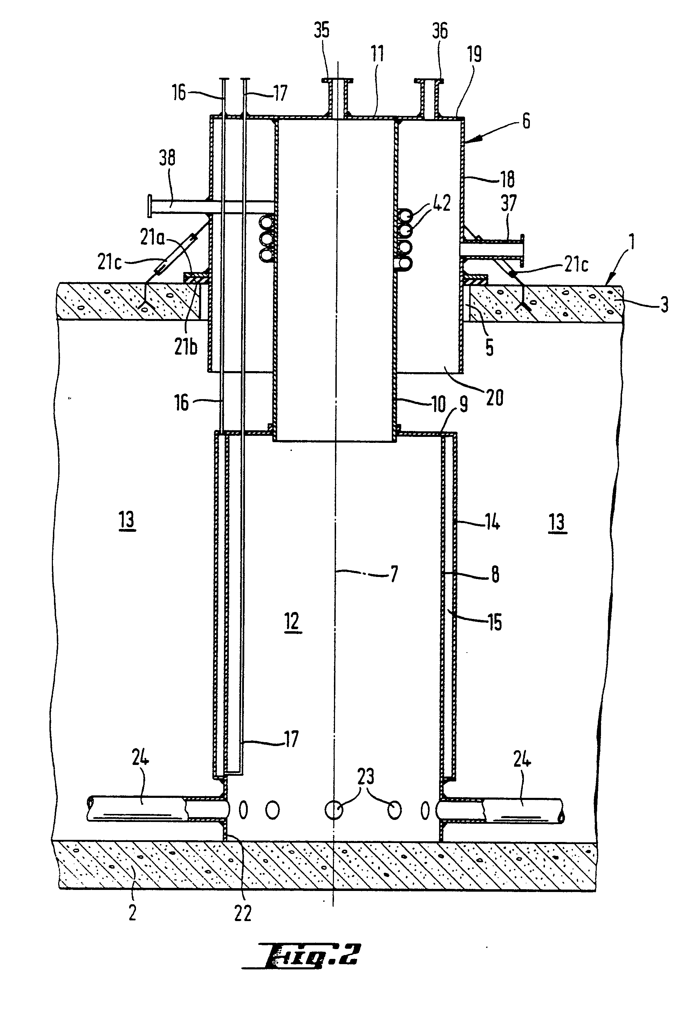

In diesem mantelfreien Stück des ersten Rohres 8 sind eine Vielzahl von Leitungen 24 — z. B. Rohre oder Schläuche — vorgesehen, durch die der Säurebildungsraum 12 mit dem Fermentationsraum 13 verbunden bzw. verbindbar ist. Diese Leitungen 24 — 12 Stück gemäß den Ausführungsformen nach Figuren 1 und 2 und 16 Stück gemäß der Ausführungsform nach Figuren 3 bis 6 pro Einsatz 6 — enden einen Endes offen in dem Fermentationsraum und zwar gemäß der Ausführungsform nach Figuren 1 und 2 in der Nähe der Behälterwandung 4 und gemäß der Ausführungsform nach Figuren 3 bis 6 etwa mittig zwischen der Achse des Behälters 1 bzw. des Einsatzes 6 und der Behälterwandung 4. Gemäß Figuren 1 und 2 enden die Leitungen 24 innenliegend an Bohrungen 23 der Wandung des Einsatzes 6, wo sie an der Wandung des Einsatzes 6 abgedichtet festgelegt, z. B. angeschweißt, sind, während gemäß der Ausführungsform nach Figuren 3 bis 6 die Leitungen 24 die Wandung des Einsatzes 6, dort abgedichtet, durchdringen und nach oben geführt werden, worauf noch eingegangen wird.

Gemäß den Ausführungsbeispielen nach Figuren 1 und 2 ist das zweite Rohr 10, welches dort etwa genauso lang ist, wie das erste Rohr 8 und dessen Durchmesser dort etwa halb so groß ist, wie der Durchmesser des ersten Rohres 8, an seinem dem Flanschring 9 abgewandten Ende durch einen Deckel 11 geschlossen. Dieser Deckel 11 ist hier lösbar aber abgedichtet an dem zweiten Rohr 10 festgelegt, sodaß bei großen Einsätzen 6, wie sie z. B. in den Figuren dargestellt sind, der Innenraum des Reaktors zu Wartungs- und Montagezwecken durch die beiden Rohre 8 und 10 hindurch sogar betreten werden kann.

Gemäß der Ausführungsform nach Figuren 3 bis 6 beträgt die axiale Länge des zweiten, kleineren Rohres 10 nur etwa einem Viertel der Länge des ersten Rohres 8. Sein Durchmesser ist allerdings größer, als der Durchmesser des zweiten Rohres 10 gemäß Figuren 1 und 2.

Das kleinere, zweite Rohr 10 ist von einem dritten Rohr 18 umgeben und mit diesem über einen Flanschring 19 verbunden.

Gemäß Figur 1 ist der zweite Flanschring 19 vom oberen Ende des zweiten Rohres 10 beabstandet und schließt mit dem oberen Ende des dritten Rohres 18 ab, welches hier wesentlich kürzer ist, als das zweite Rohr. Gemäß Figur 2 schließen das zweite und das dritte Rohr 10 und 18 oben in gleicher axialer Höhe ab und sind an ihren oberen Enden über einen Flanschring 19 miteinander verbunden. Gemäß der Ausführungsform nach Figuren 3 bis 6 ist das dritte Rohr 18 axial wesentlich länger, als das zweite Rohr 10 und zwar hier etwa 70 % länger. Der Flanschring 19 liegt hier am oberen Ende des zweiten Rohres 10.

Die Verbindung zwischen dem zweiten Rohr 10 und dem Flanschring 19 einerseits und dem dritten Rohr 18 und dem anderen Flanschring 19 andererseits ist gasdicht ausgebildet, der Flanschring 19 ist bevorzugt an den beiden Rohren 10 und 18 angeschweißt. Der Außendurchmesser dieses dritten Rohres 18 entspricht gemäß Figuren 1 und 2 etwa dem Außendurchmesser des Mantels 14 des ersten Rohres 8, während der Durchmesser des dritten Rohres 18 gemäß der Ausführungsform nach Figuren 3 bis 6 größer ist und hier etwa dem 1, 16-fachen des Durchmessers des Mantels 14 entspricht. Der Durchmesser des dritten Rohres 18 ist etwas kleiner, als der Durchmesser der Öffnung 5 im Deckel 3 des Reaktorbehälters 1. Das untere Ende des dritten Rohres 18 ist vom Mantel 14 beabstandet. Gemäß den Ausführungsbeispielen nach Figuren 1 und 2, in denen der Durchmesser des dritten Rohres 18 und des Mantels 14 in etwa übereinstimmen, ist das dritte Rohr 18 axial von dem Mantel 14 beabstandet und dieser Abstand entspricht etwa einem Viertel bis der Hälfte der Differenz zwischen den Durchmessern des dritten Rohres 18 und des zweiten Rohres 10. Gemäß der Ausführungsform nach Figuren 3 bis 6 endet das dritte Rohr 18 etwa in axialer Höhe der Oberkante des Mantels 14 bzw. etwas darunter, wobei der Abstand zwischen beiden durch den Durchmesserunterschied zwischen beiden bestimmt wird. Dadurch wird zwischen dem dritten Rohr 18 und dem Mantel 14 eine Eintrittsöffnung 20 für die ausgefaulte Biomasse gebildet, die den Behälter 1 durch den Zwischenraum zwischen zweiten und dritten Rohr 10 und 18 verläßt.

Das dritte Rohr 18 besitzt an seinem Umfang einen Auflageflansch 21a, der an diesem Rohr 18 angeschweißt ist und auf dem Deckel 3 des Reaktorbehälters 1 aufliegt. Dieser Flansch 21a muß dabei vom Außendurchmesser und von seiner Wandstärke her so ausgebildet sein, daß er den Kopf des Einsatzes 6, der gegenüber dem unteren Teil des Einsatzes 6 verschiebbar ist, tragen kann, da der Kopf des Einsatzes 6 sich über den Flansch 21a auf dem Deckel 3 abstützt. Dieses dritte Rohr 18 ist darüberhinaus gegenüber dem Deckel 3 in der Öffnung 5 gas- und flüssigkeitsdicht mittels eines Dichtungsringes 21b abgedichtet. Dieser Dichtungsring 21b liegt hier zwischen dem Deckel 3 des Reaktorbehälters 1 und dem Flanschring 21a und wird durch das Gewicht des Kopfes des Einsatzes 6 zusammengepreßt. Weiterhin sind zwischen dem Rohr 18 des Einsatzes 6 und dem Deckel 3 des Reaktorbehälters 1 noch über den Umfang des Rohres 18 verteilt mehrere Spannschlösser 21c vorgesehen, die den Kopf des Einsatzes 6 in Richtung auf den Deckel 3 des Behälters 1 verspannen.

Die Ab- bzw. Zuleitungen 16 bzw. 17 des Mantels 14 des ersten Rohres 8 (in Figuren 3 bis 6 der Übersichtlichkeit halber nicht dargestellt) dringen gasdicht geschweißt durch den Flanschring 19 und treten gemäß Figuren 1 und 2 dort aus dem Einsatz 6 und damit aus dem Reaktorbehälter 1 aus.

Bei der Ausführungsform nach Figuren 3 bis 6, bei der das dritte Rohr 18 axial wesentlich länger ist, als das zweite Rohr 10, ist nun der Flanschring 19 über das zweite Rohr 10 noch weiter nach innen auf die Achse der Rohre zu verlängert, sodaß über dem durch das zweite Rohr 10 begrenzten Raum ein dort außenliegender Ring gebildet wird. In diesem Ring sind Bohrungen 23' angeordnet, denen jeweils das andere Ende der zuvor beschriebenen Leitungen 24, deren anderes Ende im Fermentationsraum 13 endet, zugeordnet ist. Die Leitungen 24 sind an dem inneren Ring des Flanschringes 19 abgedichtet festgelegt, z. B. angeschweißt oder angeschraubt. Am inneren Ende dieses Flanschringes 19 gemäß der Ausführungsform nach Figuren 3 bis 6 ist ein viertes Rohr 25 festgelegt, welches sich von diesem Flanschring 19 nach oben erstreckt und axial unterhalb dem oberen Ende des dritten Rohres 18 endet. Der axiale Abstand zwischen dem oberen Ende des dritten Rohres 18 und des vierten Rohres 25 entspricht etwa dem horizontalen Abstand « X » zwischen dritten Rohr 18 und Mantel 14.

Unterhalb des oberen Endes des vierten Rohres 25, etwa beabstandet um den Betrag « X » ist nun im Innern des vierten Rohres 25 ein dritter Flanschring 26 angeordnet, der eine Breite von etwa « X » aufweist und an dessen innerem Ende ein sich von dort nach oben erstreckendes fünftes Rohr 27 befindet, dessen axiale Länge hier etwa dem Vierfachen der Länge « X » entspricht, weshalb dieses fünfte Rohr 27 hier zur Hälfte unterhalb und zur anderen Hälfte oberhalb des oberen Endes des dritten Rohres 18 liegt. Dieses fünfte Rohr 27 ist oben von einem Deckel 11' abgeschlossen und bildet zusammen mit diesem Deckel für den Säurebildungsraum 12 einen Gasdom, in dem das im Säurebildungsraum 12 gebildete Biogas aufgefangen wird. Der dritte Flanschring 26 weist nun — hier — zwei Bohrungen auf, in die je ein Leitungsstück 28 einer oben vergabelten Leitung 29 mündet. Das andere Ende dieser Leitung endet in der Nähe des Bodens 2 des Behälters 1 im Säurebildungsraum 12. Mittels einer in der Leitung 29 angeordneten Pumpe 30 wird die Biomasse vom Boden des Säurebildungsraumes 12 hoch in den Raum zwischen dem fünften Rohr 27, dem dritten Flanschring 26 und dem vierten Rohr 25, bzw. wenn dieser Raum überläuft, auch dem dritten Rohr 18, gepumpt.

Der Raum zwischen dem dritten Rohr 18, dem zweiten Flanschring 19 und dem vierten Rohr 25 bzw. dem fünften Rohr 27 ist in einzelne Kammern 31 aufgeteilt, deren Anzahl der Anzahl der an diesen Raum angeschlossenen Leitungen 24 entspricht. Diese einzelnen Kammern 31 sind von einem ringförmigen Deckel 32 abgeschlossen, der aus einzelnen, den Kammern 31 entsprechenden Abschnitten 32' besteht.

Bei der Ausführungsform gemäß den Figuren 3 bis 6 ist entsprechend den Kammern 31 im außerhalb des Reaktorbehälters 1 liegenden Teil des Einsatzes 6 auch der Fermentationsraumes 13 in einzelne Kammern 33 durch vertikale Abtrennungen 34, z. B. Planen aus Kunststoff, aufgeteilt. Dieses Abtrennungen reichen vom Boden 2 bis zum Deckel 3 des Fermentationsraumes einerseits und von dem Einsatz 6 zur Wandung 4 des Reaktorbehälters 1 andererseits. D. h., entsprechend den 16 Leitungen 24 und den 16 Kammern 31 des Einsatzes 6 ist der Fermentationsraum 13 in 16 Kammern 33 unterteilt. Planen aus Kunststoff oder auch aus einem anderen Material sind deswegen geeignet, da jede der 16 Kammern 33 mit Flüssigkeit gefüllt ist, die — wenn eine Kammer 33 z. B. gereinigt wird oder stillgelegt ist — aus Wasser besteht. Durch die Aufteilung des Fermentationsraumes 13 in Kammern 33 und durch das Aufteilen des oberen Teils des Einsatzes 6 in einzelne Kammern 31 kann der gesamte Reaktor besser genutzt werden.

Es ist dadurch möglich, einzelnen Abschnitten des Reaktors Materialien zuzuführen, die keiner Säurebildung mehr bedürfen, wie z. B. Weinschlempe od. dgl.. Dazu wird z. B. eine Kammer 31 im Kopf des Einsatzes 6 von den anderen Kammern 31 abgeschottet, indem man z. B. auf den dieser Kammer 31 zugeordneten Abschnitt des vierten Rohres 25, welches mit seinem oberen Ende schon als Wehr dient, eine Verlängerung aufsetzt, die verhindert, daß in den zwischen dem vierten Rohr 25 und dem fünften Rohr 27 gebildeten Raum Biomasse in diese eine Kammer 31 fließen kann. Diese eine abgeschottete Kammer 31 wird dann gesondert mit Biomasse beschickt, die durch die dieser Kammer 31 zugeordnete Leitung 24 in eine entsprechende Kammer 33 des Fermentationsraumes 13 gelangt. Diese gesondert zugeführte Biomasse kann in dieser einen Kammer 33 des Fermentationsraumes 13 mit einer abweichenden Durchflußgeschwindigkeit geführt werden. Auf diese Art und Weise kann eine einzelne Kammer 33 des Fermentationsraumes 13 auch gereinigt werden, indem man die Zuführung von Biomasse sperrt und statt dessen durch die Leitung 24 reines Wasser od. dgl. zuführt.

Im Deckel 11 des zweiten Rohres 10 (Figuren 1 und 2) bzw. im Deckel 11′ des fünften Rohres 27 (Figuren 3 bis 6) befindet sich ein erster Rohrstutzen 35, der der Abführung des im Säurebildungsraum 12 gebildeten Gases dient. Im oberen, zweiten Flanschring 19 ist ein zweiter Rohrstutzen 36 angeordnet, der in den zwischen dem zweiten und dritten Rohr 10 und 18 gebildeten Raum mündet und der Abführung des im Fermentationsraum 13 gebildeten Gases dient. Bei der Ausführungsform gemäß Figuren 3 bis 6 liegt dieser zweite Rohrstutzen 36 zum Teil in einer zwischen dem dritten Rohr 18 und dem vierten Rohr 25 gebildeten Kammer 31 und durchdringt aber gleich oberhalb des zweiten Flanschringes 19 das dritte Rohr 18, um außerhalb des Einsatzes 6 zu enden.

Im mittleren Bereich des dritten Rohres 18, außerhalb des Reaktorbehälters 1, ist ein horizontal liegender dritter Rohrstutzen 37 angeordnet, der auch in den zwischen dem zweiten und dritten Rohr 10 und 18 gebildeten Raum mündet und durch den die ausgefaulte Biomasse abgeführt wird.

Gemäß Figur 1 durchdringt ein vierter Rohrstutzen 38 den Deckel 11 des zweiten Rohres 10 in vertikaler Richtung. Das untere Ende dieses vierten Rohrstutzens 38 steht nach unten etwa bis zur Achse des dritten Rohrstutzens 37 vor. Dieser vierte Rohrstutzen 38 mündet in den Säurebildungsraum 12 und dient der Zufuhr frischer Biomasse. Schlempe und auch Molkereiabfälle haben oft eine Temperatur von etwa 85 °C. Beim Einleiten in den Reaktor beträgt die Temperatur immer noch etwa 60 °C. Um diese frische Biomasse von der Zuführ-Temperatur von z. B. 60 °C auf die optimale Verfahrenstemperatur von 34 °C abzukühlen, ist gemäß Figur 1 etwa in Höhe des dritten Rohrstutzens 37 das zweite Rohr 10 von einem Mantel 39 umgeben. In dem Raum zwischen diesem Mantel 39 und dem zweiten Rohr 10 strömt eine Kühlflüssigkeit, die über Leitungen 40 und 41 zu- bzw. abgeführt wird.

Gemäß einer anderen, nicht dargestellten Ausführungsform mündet der dem Beschicken des Reaktors dienende vierte Rohrstutzen 38 durch den Deckel 11 des zweiten Rohres 10 in den Säurebildungsraum 12, wobei sein unteres Ende von diesem Deckel 11 beabstandet sein kann.

Gemäß Figur 2 wird die frische Biomasse durch einen vierten Rohrstutzen 38 zugeführt, der oberhalb der Achse des dritten Rohrstutzens 37 für die Abführung der ausgefaulten Biomasse und auf der in Bezug auf die Achse 7 des Einsatzes 6 gegenüberliegenden Seite liegt. Dieser vierte Rohrstutzen 38 durchdringt das dritte Rohr 18 des Einsatzes 6 und mündet in eine Rohrschlange 42, die sich in dem von dem zweiten Rohr 10 und dem dritten Rohr 18 gebildeten Raum um das zweite Rohr 10 in mehreren Schleifen windet und dann in den von dem zweiten Rohr 10 umschlossenen Raum, dem oberen Teil des Säurebildungsraums 12, mündet. Dadurch wird die frisch zugeführte Biomasse im wesentlichen nur durch die abzuführende, ausgefaulte und kühlere Biomasse gekühlt.

Gemäß der Ausführungsform nach Figuren 3 bis 6 durchdringt ein vierter Rohrstutzen 38 die Wandung des fünften Rohres 27 und ist dann in der Achse des Einsatzes 6 nach unten abgewinkelt. Dieser vierte Rohrstutzen 38 endet knapp oberhalb der vergabelten Leitung 29. Eine Kühlung der frischzugeführten Biomasse erübrigt sich hier meistens, da hier durch den oberen Bereich des Säurebildungsraumes 12 die Leitung 29 einerseits und die Leitungen 24 andererseits geführt werden, in denen die schon im Säurebildungsraum reagierte Biomasse geführt wird.

Der gesamte Einsatz 6 läßt sich fertigmontiert z. B. mittels eines Kranes in die Öffnung 5 des Reaktordeckels 3 einsetzen und auch z. B. zu Wartungszwecken wieder herausnehmen, wobei der untere, im wesentlichen aus dem ersten Rohr 8 und dem Mantel 14 dieses Rohres 8 und dem ersten Flanschring 9 bestehende Teil des Einsatzes 6 getrennt von dem oberen Teil, dem Kopfteil des Einsatzes 6 bewegt werden kann.

Der Einsatz 6 weist an seinem unteren, vom Mantel 14 freien Ende 22 die das erste Rohr 8 durchdringenden Bohrungen 23 auf, über den Umfang verteilt 12 Bohrungen gemäß Figuren 1 und 2 und 16 Bohrungen gemäß Figuren 3 bis 6, die in ebensoviele Leitungen 24 münden. Die Leitungen 24 liegen hier auf Füßen 45, die sich auf dem Boden 2 abstützen, auf.

Gemäß Figuren 1 und 2 enden diese Leitungen 24 im Bereich der Behälterwandung 4. Die Leitungen 24 bilden gemäß Figuren 1 und 2 eine direkte Verbindung zwischen dem Säurebildungsraum 12 und dem Fermentationsraum 13 und sorgen gleichzeitig dafür, daß die aus dem Säurebildungsraum 12 kommende Biomasse gleichmäßig über den Fermentationsraum 13 verteilt und dort in die der Eintrittsöffnung 20 des Einsatzes 6 entfernteste Lage gebracht werden.

Der Fermentationsraum 13 kann nun zumindest zum Teil mit Füllgut aus großen Partikeln eines offenporigen Materials gefüllt sein. Dieses Füllgut weist je nach zu verarbeitender Biomasse einen Durchmesser zwischen 200 mm und 10 mm auf.

Bei der Ausführungsform gemäß den Figuren 3 bis 6 ist der Fermentationsraumes 13 in 16 Kammern 33 aufgeteilt. Ist hier Füllgut vorgesehen, so besteht dieses hier aus Partikeln, die etwa einen Durchmesser von 100 mm bis 200 mm aufweisen. Wird eine Kammer 33 des Fermentationsraumes 13 immer für ein spezielles Material, z. B. nur für Schlempe, benutzt, so kann das Füllgut in dieser Kammer 33 auch einen kleineren Partikeldurchmesser aufweisen, der dann z. B. nur 10 mm bis 20 mm beträgt.

Je eine Leitung 24 mündet in eine einzelne Kammer 33, wie oben schon beschrieben, und liegt z. B. unmittelbar auf einem Teil des Füllgutes auf, durch das es abgestützt wird. Die Leitungen 24 enden hier weit beabstandet von der Wandung des Behälters 1 und ragen von dem Einsatz 6 eine Länge in den Fermentationsraum 13 hinein, die etwa einem Drittel des Abstandes zwischen dem Einsatz 6 und der Behälterwandung 4 beträgt.

Da der unterste Rand des dritten Rohres 18, durch den die abgebaute Biomasse aus dem Reaktorbehälter 1 geführt wird, unterhalb des Behälterdeckels 3 liegt, fängt sich unter dem Behälterdeckel 3 im Fermentationsraum 13 gebildetes Biogas. Andererseits fließt die ausgefaulte Biomasse oberhalb des Behälterdeckels 3 aus dem am Einsatz 6 angeordneten, dritten Rohrstutzen 37; die Flüssigkeitsniveaus des Säurebildungsraumes 12 und des Fermentationsraumes 13 liegen also über dem Behälterdeckel 3. Der Behälterdeckel 3 weist Gasdome 52 auf, deren Oberkante sich hier etwa in Höhe des dem Abführen der ausgefaulten Biomasse dienenden, dritten Rohrstutzens 37 befinden bzw. knapp darunter außerhalb des Reaktorbehälters 1 liegen und an deren oberen Ende Leitungen 53 zum Abführen des Biogases angeordnet sind.

Während bei der Ausführungsform im rechten Teil der Figur 1 und gemäß Figur 2 und bei der Ausführungsform nach Figuren 3 bis 6 mehrere Gasdome 52 auf einem bestimmten Radius um den Einsatz 6 angeordnet sind, sind bei der anderen Ausführungsform im linken Teil der Figur 1 auf mehreren Radien Gasdome 52 angeordnet.

Die aus den Gasdomen 52 herausführenden Gas-Leitungen 53 können in eine nicht dargestellte Ringleitung münden, in die auch die mit dem zweiten Rohrstutzen 36 verbundene Gas-Leitung mündet. Diese Gasleitungen 53 können aber auch, wie in der Ausführungsform nach Figuren 3 bis 6 gezeigt, zu zwei halbringförmigen Leitungen 54 führen, die ihrerseits oben in den zwischen dem zweiten Rohr 10 und dem dritten Rohr 18 gebildeten Raum oberhalb des dem Abführen der ausgefaulten Biomasse dienenden dritten Rohrstutzen 37 führen.

Für einen Reaktor mit einem Füllvolumen von 1 000 m³ bis 1 200 m³, der dann z. B. einen Innendurchmesser von 12 m bis 16 m und eine lichte Höhe von 6 m bis 10 m aufweist, wird ein Einsatz 6 verwendet, dessen axiale Länge etwa 9 m bis 14 m beträgt, während die Öffnung 5 im Reaktordeckel 3 und damit auch der Außendurchmesser des dritten Rohres 18 etwa 2,5 m bis 3,5 m beträgt. Der Reaktordeckel 3 weist hier eine Wandstärke von etwa 0,4 m bis 0,5 m auf. Der Innendurchmesser des kleineren, zweiten Rohres 10 beträgt dann etwa 1,3 m bis 1,6 m bzw. 2 m. Wird der Deckel 11 des zweiten Rohres 10, der als Mannlochöffnung dient, geöffnet, so kann der Reaktor durch den Einsatz 6 hindurch betreten werden.

Wie schon oben gesagt wird der Reaktor durch den vierten Rohrstutzen 38 hindurch beschickt. Die Biomasse gelangt dadurch in den Innenraum des zweiten Rohres 10 und wird in diesem Eintrittsbereich gekühlt. Von dort fällt die Biomasse nach unten in den Innenraum des größeren, ersten Rohres 8. Beide Innenräume zusammen bilden den Säurebildungsraum 12. In diesem Säurebildungsraum 12 verweilt die Biomasse mehrere Stunden, bevor sie gemäß den Ausführungsformen nach Figuren 1 und 2 durch die Bohrungen 23 am unteren Ende 22 des ersten Rohres 8 und durch die Rohre oder Schläuche 24 in den Fermentationsraum 13 gelangt, der von der Wandung 4 des Reaktorbehälters 1 einerseits und von dem ersten und zweiten Rohr 8 und 10 andererseits begrenzt wird. Bei der Ausführungsform nach Figuren 3 bis 6 wird die Biomasse durch die Leitung 29 mittels der Pumpe 30 in den Kopf des Einsatzes 6 gepumpt und läuft dann in die Kammern 31 über, wenn diese nicht gesperrt sind. Andererseits können diese Kammern 31 auch direkt beschickt werden. Aus diesen Kammern 31 läuft die Biomasse durch die Leitungen 24 in die Kammern 33 des Fermentationsraumes 13. Die zwischen dem ersten Rohr 8 und dem Mantel 14 strömende Wärmeflüssigkeit hält die Biomasse auf der optimalen Verfahrenstemperatur. Da bei dem Verfahren Wärme verbraucht wird, ist eine Wärmenachfuhr notwendig.

Im Fermentationsraum 13 steigt insbesondere das entstandene Biogas nach oben. Das Biogas sammelt sich zum einen unterhalb des Behälterdeckels 3 und in den Domen 52 und zum anderen auch in dem von dem zweiten und dritten Rohr 10 und 18 und dem Flanschring 19 gebildeten Raum, in den hier auch die von den Gasdomen 52 kommenden Leitungen 53 bzw. 54 führen und wird durch den zweiten Rohrstutzen 36 abgeführt. Die Gasabführung erfolgt unter Druck, wofür eine Wasservorlage benutzt wird, die ähnlich ausgebildet ist, wie die für die Abfuhr der ausgefaulten Biomasse in Figur 3 gezeigte Wasservorlage 55. Die ausgefaulte Biomasse wird durch den dritten Rohrstutzen 37 abgeführt, der Auslauf erfolgt über einen in Figuren 1 und 2 nicht dargestellten Überlauf bzw. über die in Figur 3 gezeigte Wasservorlage 55.

Im Säurebildungsraum 12 entstehende Gase, im wesentlichen Kohlendioxid, werden im durch das zweite Rohr 10 und dessen Deckel 11 gebildeten Dom aufgefangen und über den ersten Rohrstutzen 35 abgeführt.

Die Verweilzeit der Biomasse in dem Reaktor beträgt z. B. 24 Stunden. Dann wird z. B. stündlich diskontinuierlich 1/24 des Füllvolumens in den Reaktor gepumpt, wodurch die gleiche Menge ausgefaulte Biomasse aus dem Reaktor ausläuft. Die Füllhöhe bleibt dabei im Reaktor immer konstant. Aus Sicherheitsgründen ist oberhalb des vierten Rohrstutzens 38 im Innern des zweiten Rohres 10 ein nicht dargestellter Schwimmerschalter angeordnet, der im Notfall, wenn z. B. der Auslauf verstopft sein sollte, die Zufuhr frischer Biomasse selbsttätig stoppt. Im Raum zwischen dem zweiten und dem dritten Rohr 10 und 18 kann oberhalb des Auslaufes ein weiterer Schwimmerschalter vorgesehen sein.

Der Faulungsprozeß wird unter einem leichten Überdruck von etwa 300 mbar, maximal 1 bar, durchgeführt. Der Reaktordeckel 3 liegt damit nicht nur auf der Biomasse auf, er wird durch den Überdruck zusätzlich nach oben gedrückt.

Das Füllgut aus großen Partikeln besteht aus einem Material welches Poren mit einem Durchmesser aufweist, der zwischen 0,8 μm und 220 μm, bevorzugt zwischen 1 μm und 10 μm beträgt. Diese Bedingungen werden z. B. von offenporigem Steingut, wie Schamott oder Ziegel. erfüllt, wobei insbesondere Steingutbruch zur Anwendung gelangt. Wesentlich ist weiterhin, daß das aufgeschüttete Füllgut ausreichend große Zwischenräume zwischen den einzelnen Partikeln freiläßt, damit auch in der Biomasse enthaltene Feststoffteilchen zwischen den Partikeln hindurchströmen können. Da diese Teilchen allmählich abgebaut werden, kann die Körnung über die Länge einer Führungsbahn kleiner werden, wobei auch die Zwischenräume zwischen den Partikeln kleiner werden. Je nach zu verarbeitender Biomasse können deshalb die Partikel eine andere Größe aufweisen. Partikel mit einem Durchmesser von 200 mm in der Nähe des Austrittes aus den Leitungen 24 bei größeren Feststoffteilchen sind somit genauso vorstellbar, wie Partikel mit einem Durchmesser von 10 mm und kleiner in der Nähe der Eintrittsöffnung 20 des Kopfes des Einsatzes 6 und einer Ausgangsbiomasse, die nur kleine oder kleinste Feststoffteilchen enthält. Bei der Ausführungsform nach Figuren 3 bis 6 werden auf den Boden 2 des Reaktorbehälters 1 hauptsächlich Partikel mit größerem Durchmesser abgelegt. In einzelnen Kammern 33, in denen z. B. immer nur Gülle eingefüllt wird, können auch Füllgutpartikel mit einem Durchmesser von etwa 10 mm vorhanden sein, da nur Flüssigkeiten verarbeitet werden.

Wenngleich oben verschiedene Ausführungsbeispiele nebeneinander beschrieben wurden, kann der Fachmann einzelne an einem Ausführungsbeispiel beschriebene Maßnahmen auch an anderen Ausführungsbeispielen durchführen. Bestimmte Einzelheiten des Reaktors können auch anders ausgebildet sein, als oben beschrieben.

Durch die Ausführungsform des Reaktors nach Figuren 3 bis 6 wird auch verbessert sichergestellt, daß keine Methanbakterien vom Fermentationsraum 13 in den Säurebildungsraum 12 gelangen können.

Bezugszeichenliste

1 Reaktorbehälter
2 Boden des Behälters 1
3 Deckel des Behälters 1
4 Wandung des Behälters 1
5 Öffnung des Deckels 3
6 Einsatz
7 Achse des Einsatzes 6
8 erstes Rohr des Einsatzes 6
9 erster Flanschring des Einsatzes 6
10 zweites Rohr des Einsatzes 6
11 Deckel des zweiten Rohres 10
11' Deckel des fünften Rohres 27
12 Säurebildungsraum
13 Fermentationsraum
14 Mantel des ersten Rohres 8
15 Raum zwischen dem Rohr 8 und dem Mantel 14
16 Leitung
17 Leitung
18 drittes Rohr des Einsatzes 6
19 zweiter Flanschring des Einsatzes 6
20 Eintrittsöffnung
21a Auflageflansch
21b Dichtungsring
21c Spannschloß
22 unteres Ende des ersten Rohres 8
23 Bohrung im ersten Rohr 8
23' Bohrung
24 Leitungen, (z. B. Rohre oder Schläuche)
25 viertes Rohr des Einsatzes 6
26 dritter Flanschring des Einsatzes 6
27 fünftes Rohr des Einsatzes 6
28 Leitungsstück der Leitung 29
29 Leitung
30 Pumpe in der Leitung 29
31 Kammer (oben)
32 Deckel
32' Abschnitt des Deckels 32 (entsprechend den Kammern 31)
33 Kammer im Fermentationsraum
34 vertikale Abtrennung
35 erster Rohrstutzen
36 zweiter Rohrstutzen
37 dritter Rohrstutzen
38 vierter Rohrstutzen
39 Mantel des zweiten Rohres 10
40 Zuleitung
41 Ableitung
42 Rohrschlange
45 Fuß
52 Gas-Dom
53 Gas-Leitung
54 Gas-Leitung
55 Wasservorlage
X Abstand

## Patentansprüche

1. Biohochleistungsdurchlaufreaktor, insbesondere zur anaeroben Aufbereitung pflanzlicher und tierischer Biomasse, auch von Abfall, aus anorganischen Materialien mit relativ hohem Molekulargewicht, bei dem in einem Reaktorraum die hochmolekularen organischen Materialien durch Bakterienstämme in organische Materialien mit niedrigem Molekulargewicht und diese in niedrige organische Säuren unter Bildung von Wasserstoff, Kohlendioxid und Schwefelwasserstoff in Essigsäure übergeführt werden, dann die entstehenden Substanzen in Methan und Kohlendioxid umgewandelt werden, wobei in dem Reaktorraum eine Aufteilung von Säurephase und Methanphase vorzugsweise im umgekehrten Verhältnis zu den Abbauraten der Prozeßstufen Säurebildung und Methanbildung erfolgt, wodurch in dem abgeteilten Raum für die Säurephase die abgebauten Produkte nach unten sinken und durch eine oder mehrere offene Verbindungen in den Fermentationsraum für die Methanbildung gelangen, dadurch gekennzeichnet, daß ein von dem Reaktorbehälter trennbarer, in den Deckel des Reaktorbehälters einhängbarer Einsatz (6) vorgesehen ist, der eine rohrförmige Einrichtung zum Aufteilen des Faulraumes in einen Säurebildungsraum (12) und einen Fermentationsraum (13) und Zu- und Abführeinrichtungen für die Biomasse und die entstandenen Gase aufweist.

2. Reaktor nach Anspruch 1, dadurch gekennzeichnet, daß der Einsatz (6) im wesentlichen aus mehreren, konzentrisch zueinander angeordneten Rohren (8, 10, 18) besteht, die über Flanschringe (9, 19) miteinander verbunden sind.

3. Reaktor nach Anspruch 2, dadurch gekennzeichnet, daß am oberen Ende eines ersten Rohres (8) ein sich von dort nach innen erstreckender erster Flanschring (9) angeordnet ist, an dessen innerem Rand das untere Ende eines zweiten Rohres (10) festgelegt ist.

4. Reaktor nach einem der vorhergehenden Ansprüche, dadurch gekennzeichnet, daß das erste Rohr (8) fast über seine gesamte Länge von einem Mantel (14) umgeben ist und zusammen mit diesem einen hohlzylinderförmigen Raum (15) umgibt, in den zwei Leitungen (16, 17) zum Zu- bzw. Abführen einer Wärmeflüssigkeit münden.

5. Reaktor nach Anspruch 3, dadurch gekennzeichnet, daß das zweite, kleinere Rohr (10) von einem dritten Rohr (18) umgeben ist und diese beiden Rohre (10, 18) über einen zweiten Flanschring (19) miteinander verbunden sind.

6. Reaktor nach dem vorhergehenden Anspruch, dadurch gekennzeichnet, daß der Durchmesser dieses dritten Rohres (18) mindestens die Größe des Außendurchmessers des Mantels (14) des ersten Rohres (8) aufweist.

7. Reaktor nach einem der beiden vorhergehenden Ansprüche, dadurch gekennzeichnet, daß das untere Ende des dritten Rohres (18) von dem oberen Ende des Mantels (14) beabstandet ist.

8. Reaktor nach einem der Ansprüche 5 bis 7, dadurch gekennzeichnet, daß auf der dem ersten Rohr (8) abgewandten Seite des zweiten Flanschringes (19) an diesem in dem Abführen des Biogases dienender zweiter Rohrstutzen (36) angeordnet ist.

9. Reaktor nach einem der Ansprüche 5 bis 8, dadurch gekennzeichnet, daß etwa im mittleren Bereich des dritten Rohres (18) zwischen dem zweiten Flanschring (19) und dem Deckel (3) des Reaktorbehälters (1) ein an diesem dritten Rohr (18) befestigter und nach außen gerichteter, in dieses dritte Rohr (18) mündender, dem Abführen der ausgefaulten Biomasse dienender dritter Rohrstutzen (37) angeordnet ist.

10. Reaktor nach einem der vorhergehenden Ansprüche, dadurch gekennzeichnet, daß das erste und das zweite Rohr (8, 10) den Säurebildungsraum (12) umschließen.

11. Reaktor nach einem der vorhergehenden Ansprüche, dadurch gekennzeichnet, daß die gemeinsame Achse (7) der drei Rohre (8, 10, 18) des Einsatzes (6) im eingebauten Zustand vertikal ausgerichtet ist und in der Achse des kreiszylinderförmig ausgebildeten Reaktorbehälters (1) angeordnet ist.

12. Reaktor nach einem der vorhergehenden Ansprüche, dadurch gekennzeichnet, daß der Einsatz (6) mit dem unteren Ende (22) seines ersten Rohres (8) auf dem Boden (2) des Reaktorbehälters (1) aufsteht und das zweite und das dritte Rohr (10, 18) durch die Öffnung (5) des Deckels (3) des Reaktorbehälters (1) hindurchdringen und die dem Zu- und Abführen der Biomasse und dem Abführen des gebildeten Biogases dienenden Rohrstutzen (35, 36, 37, 38) außerhalb des Reaktorbehälters liegen.

13. Reaktor nach einem der vorhergehenden Ansprüche, dadurch gekennzeichnet, daß der Fermentationsraum (13) durch eine Vielzahl von vertikalen Abtrennungen (34) in einzelne Kammern (33) aufgeteilt ist und in jede dieser Kammern (33) eine Leitung (24) mündet und im Deckel (3) des Reaktorbehälters (1) für jede Kammer (33) ein Gasdom (52) zur Abführung des gebildeten Biogases angeordnet ist.

14. Reaktor nach einem der vorhergehenden Ansprüche, dadurch gekennzeichnet, daß er ein Füllgut aus offenporigem Steingut, z. B. Schamott oder Ziegel, aufweist.

15. Reaktor nach einem der vorhergehenden Ansprüche, dadurch gekennzeichnet, daß die Poren des Füllgutes einen Durchmesser aufweisen, der zwischen 0,8 μm und 220 μm, vorzugsweise zwischen 1 m und 10 m beträgt.

## Claims

1. High-performance run-through biochemical reactor, in particular for anaerobic treatment of vegetable and animal bio-masses, also of refuse, from inorganic materials of relatively high molecular weight, in which the high-molecular organic materials are converted in a reactor chamber by bacteria parent stocks into organic materials of lower molecular weight and these are converted into lower organic acids with the formation of hydrogen, carbon dioxide and hydrogen sulphide in acetic acid, then the substances which are produced are converted into methane and carbon dioxide, a division of acid phase and methane phase taking place in the reactor chamber preferably in inverse relationship to the decomposition rates of the process stages acid formation and methane formation, whereby in the divided chamber for the acid phase the decomposed products sink downwards and pass through one or more open connections into the fermentation chamber for forming the methane, characterised in that there is provided an insert (6) which is separable from the reactor vessel and can be hung in the cover of the reactor vessel and which comprises a tubular device for dividing up the decomposition chamber into an acid formation chamber (12) and a fermentation chamber (13) and feed and discharge devices for the biomass and the gases which are produced.

2. Reactor according to claim 1, characterised in that the insert (6) comprises substantially a number of concentrically arranged tubes (8, 10, 18) which are connected together through flange rings (9, 19).

3. Reactor according to claim 2, characterised in that a first flange ring (9) is arranged at the upper end of a first tube (8) and extends inwards from there, and the lower end of a second tube (10) is secured to the inner edge of the ring.

4. Reactor according to one of the foregoing claims, characterised in that the first tube (8) is surrounded over almost its entire length by a jacket (14) and together with the latter it defines a hollow cylindrical space (15) into which two pipes (16, 17) open for the supply and removal of a heating liquid.

5. Reactor according to claim 3, characterised in that the second, smaller, tube (10) is surrounded by a third tube (18) and these two tubes (10, 18) are connected together through a second flange ring (19).

6. Reactor according to the foregoing claim, characterised in that the diameter of this third tube (18) has at least the magnitude of the outside diameter of the jacket (14) of the first tube (8).

7. Reactor according to one of the two foregoing claims, characterised in that the lower end of the third tube (18) is spaced away from the upper end of the jacket (14).

8. Reactor according to one of claims 5 to 7, characterised in that on that side of the second flange ring (19) which faces away from the first tube (8) there is arranged on it a second pipe connection (36) serving to conduct away the biogas.

9. Reactor according to one of claims 5 to 8, characterised in that about in the central region of the third tube (18) between the second flange ring (19) and the cover (3) of the reactor vessel (1) there is arranged a third pipe connection (37) secured to this third tube (18) and outwardly directed, opening into this third tube (18) and serving to conduct away the spent bio-mass.

10. Reactor according to one of the foregoing claims, characterised in that the first and the second tube (8, 10) enclose the acid formation chamber (12)

11. Reactor according to one of the foregoing claims, characterised in that the common axis (7) of the three tubes (8, 10, 18) of the insert (6) in the installed condition is vertical and is arranged on the axis of the round cylindrically shaped reactor vessel (1).

12. Reactor according to one of the foregoing claims, characterised in that the insert (6) stands with the lower end (22) of its first tube (8) on the floor (2) of the reactor vessel (1) and the second and third tubes (10, 18) project through the opening (5) in the cover (3) of the reactor vessel (1) and the pipe connections (35, 36, 37, 38) serving for the supply and removal of the biomass and the removal of the biogas which is generated lie outside the reactor vessel.

13. Reactor according to one of the foregoing claims, characterised in that the fermentation chamber (13) is divided up by a number of vertical partitions (34) into individual chambers (33) and into each of these chambers (33) there opens a pipe (24) and in the cover (3) of the reactor vessel (1) for each chamber (33) a gas dome (52) is arranged for conducting away the biogas which is generated.

14. Reactor according to one of the foregoing claims, characterised in that it has a filling material of open-pore stone material, e. g. chamotte or tiles.

15. Reactor according to one of the foregoing claims, characterised in that the pores of the filling material have a diameter which amounts to between 0.8 μm and 220 μm, preferably between 1 m and 10 m.

**Revendications**

1. Réacteur biologique à déplacement de haut rendement, en particulier pour le traitement anaérobie de masses biologiques animales et végétales, également de déchets de matières minérales d'un poids moléculaire relativement élevé, dans lequel les matières organiques à poids moléculaire élevé sont transformées, dans une cuve de réacteur, par des souches de bactéries, en matières organiques à bas poids moléculaire et celles-ci sont transformées en acides organiques inférieurs, tout en formant de l'hydrogène, du dioxyde de carbone et de l'acide sulfhydrique dans de l'acide acétique, les substances produites sont ensuite converties en méthane et dioxyde de carbone, une séparation de la phase acide et de la phase méthane ayant lieu dans la cuve du réacteur de préférence selon un rapport inverse relativement aux taux de dégradation des stades de procédé formation d'acide et formation de méthane, de sorte que les produits dégradés s'enfoncent vers le bas dans la chambre partagée pour la phase acide et parviennent, par un ou plusieurs raccordements ouverts, à la chambre de fermentation pour la formation de méthane, caractérisé en ce qu'on a prévu un élément d'insertion (6) séparable de la cuve du réacteur, accrochable au couvercle de la cuve du réacteur et comprenant un dispositif tubulaire pour la division de la chambre de décomposition en une chambre de formation d'acide (12) et une chambre de fermentation (13), ainsi que des systèmes d'amenée et d'évacuation de la masse biologique et des gaz produits.

2. Réacteur selon la revendication 1, caractérisé en ce que l'élément d'insertion (6) se compose en substance de plusieurs tubes (8, 10, 18) disposés concentriquement l'un dans l'autre et reliés les uns aux autres par des bagues bridées (9, 19).

3. Réacteur selon la revendication 2, caractérisé en ce qu'on a disposé, à l'extrémité supérieure d'un premier tube (8), une première bague bridée (9) se prolongeant vers l'intérieur depuis ladite extrémité et au bord interne de laquelle est fixée l'extrémité inférieure d'un deuxième tube (10).

4. Réacteur selon l'une quelconque des revendications 1 à 3, caractérisé en ce que le premier tube (8) est entouré, presque sur sa longueur entière, d'une enveloppe (14) et enferme, conjointement avec celle-ci, un espace (15) d'une forme cylindrique creuse où débouchent deux conduits (16, 17) pour l'amenée ou l'évacuation d'un liquide chaud.

5. Réacteur selon la revendication 3, caractérisé en ce que le deuxième tube (10) plus petit est entouré d'un troisième tube (18) et ces deux tubes (10, 18) sont reliés l'un à l'autre par une deuxième bague bridée (19).

6. Réacteur selon l'une quelconque des revendications 1 à 5, caractérisé en ce que le diamètre de ce troisième tube (18) a au moins la grandeur du diamètre extérieur de l'enveloppe (14) du premier tube (8).

7. Réacteur selon l'une quelconque des revendications 1 à 6, caractérisé en ce que l'extrémité inférieure du troisième tube (18) est distante de l'extrémité supérieure de l'enveloppe (14).

8. Réacteur selon l'une quelconque des revendications 5 à 7, caractérisé en ce qu'une deuxième tubulure (36) servant à l'évacuation du gaz biologique est disposée sur la deuxième bague bridée (19) du côté de celle-ci éloigné du premier tube (8).

9. Réacteur selon l'une quelconque des revendications 5 à 8, caractérisé en ce qu'une troisième tubulure (37) servant à l'évacuation de la masse biologique décomposée, débouchant dans le troisième tube (18), orientée vers l'extérieur et fixée sur ledit troisième tube (18) est disposée approximativement dans la zone médiane de ce troisième tube (18) entre la deuxième bague bridée (19) et le couvercle (3) de la cuve (1) du réacteur.

10. Réacteur selon l'une quelconque des revendications 1 à 9, caractérisé en ce que le premier et le deuxième tube (8, 10) enferment la chambre de formation d'acide (12).

11. Réacteur selon l'une quelconque des revendications 1 à 10, caractérisé en ce que l'axe commun (7) des trois tubes (8, 10, 18) de l'élément d'insertion (6) est aligné verticalement à l'état monté et est disposé dans l'axe de la cuve 1 du réacteur réalisée sous une forme cylindrique.

12. Réacteur selon l'une quelconque des revendications 1 à 11, caractérisé en ce que l'élément d'insertion (6) est monté en posant l'extrémité inférieure (22) de son premier tube (8) sur le fond (2) de la cuve (1) du réacteur, en ce que le deuxième et le troisième tube (10, 18) passent par l'ouverture (5) du couvercle (3) de la cuve (1) du réacteur et en ce que les tubulures (35, 36, 37, 38) servant à l'amenée et à l'évacuation de la masse biologique et à l'évacuation du gaz biologique produit se situent à l'extérieur de la cuve du réacteur.

13. Réacteur selon l'une quelconque des revendications 1 à 12, caractérisé en ce que la chambre de fermentation (13) est divisée en différents compartiments (33) par un grand nombre de parois de séparation verticales (34) et en ce qu'un conduit (24) aboutit à chacun de ces compartiments (33) et un dôme à gaz (52) pour l'évacuation du gaz biologique formé est disposé dans le couvercle (3) de la cuve (1) du réacteur pour chaque compartiment (33).

14. Réacteur selon l'une quelconque des revendications 1 à 13, caractérisé en ce qu'il comprend un produit de remplissage à pores ouverts du type argileux, tel que de la chamotte ou des briques.

15. Réacteur selon l'une quelconque des revendications 1 à 14, caractérisé en ce que les pores du produit de remplissage ont un diamètre compris entre 0,8 $\mu$m et 220 $\mu$m, de préférence entre 1 $\mu$m et 10 $\mu$m.

Fig.1

**Fig.2**

**Fig. 3**

Fig. 4.

# Fig. 5

EP 0 121 729 B1

**Fig. 6**

EP 0 121 729 B1